# EUROPEAN PATENT SPECIFICATION

(11) **EP 0 515 554 B1**
(45) Date of publication and mention of the grant of the patent: **07.05.1997**
(21) Application number: 91905288.6
(22) Date of filing: 13.02.1991
(51) Int. Cl.: C12P 21/06, B01D 61/14, G01N 33/53, C12Q 1/00, C07K 1/12

(54) **METHOD FOR GENERATING AND SCREENING USEFUL PEPTIDES**
METHODE ZUR HERSTELLUNG UND PRÜFUNG VON VERWENDBAREN PEPTIDEN
PROCEDE DE PRODUCTION ET DE TRI DE PEPTIDES UTILES

(30) Priority: 14.02.1990 US 480865
(43) Date of publication of application: 02.12.1992
(73) Proprietor: RECEPTOR LABORATORIES, INC., Chicago, IL 60612-3501 (US)
(72) Inventor: VENTON, Duane, L., Lombard, IL 60148 (US)
(74) Representative: Andersen, Henrik Rastrup
(86) International application number: US9100891
(87) International publication number: WO9112331

(56) References cited:
- EP-A- 0 047 879
- EP-A- 0 088 398
- AU-B- 576 750
- US-A- 3 649 457
- US-A- 3 843 443
- WO 87/01374
- Hill et al (1965) Adv. in Prot. Chem. Vol.20, p. 37, 63-107
- Jakoby et al (1971) Methods in Enzymology XXII p. 22-27, 34

## Description

### 1. Field of the Invention

The invention relates to novel methods for generating and screening peptides which are useful for a variety of applications. Small peptides (2-15 amino acids) are capable of possessing unique spatial and thermodynamic properties which allow them to bind specifically to particular proteins. The binding of these peptides to such proteins can transiently or permanently eliminate or activate the function of those proteins. Thus a peptide which binds to, and eliminates the function of a coat protein of a virus could serve as an effective pharmacologic agent against that virus. Similarly, a peptide which binds to and activates a receptor for taste or olfaction could serve as a useful active agent for flavoring or perfumes. There is considerable interest in the pharmaceutical industry in the development of such useful peptides. The major difficulty is that the production and screening of individual peptides is inefficient and labor intensive. Peptides must be made individually and tested for their ability to bind the protein of interest. Because of the difficulty of making and screening each individual peptide, it is not possible to screen large numbers of peptides. There is, therefore, a need for methods which allow for rapid and efficient screening of a large number of peptides. An ideal system would provide for the simultaneous synthesis and screening of every possible peptide of a particular size.

### 2. Information Disclosure

A variety of proteolytic enzymes, including trypsin, chymotrypsin, pepsin, papain, bromelain, thermolysin and S. griseus proteinase, hydrolyze peptide bonds. The rate of hydrolysis is influenced by the choice of enzymes and the residues adjacent to the peptide bond. S. griseus proteinase, papain, and the subtilisin enzymes are known to have broad substrate specificity, but for total enzymatic hydrolysis, the use of a mixture of enzymes is preferred. Hill, "Hydrolysis of Proteins", in Advances in Protein Chemistry, 20: 37, 89-90 (Anfisen, Jr., et al., eds., 1965).

In 1937, it was demonstrated that a proteolytic reaction could be reversed. Soon thereafter, proteases were used for both stepwise and fragment condensation synthesis of peptides of predetermined sequence. Since the enzymes differed in terms of which peptide bonds would be formed, several different enzymes had to be used in succession to make oligopeptides. Unfortunately, enzymatic chain elongation could endanger preexisting bonds. See Sakina, et al., Int. J. Peptide Protein Res., 31: 245-52 (1988); Kullman, Proc. Nat. Acad. Sci. (U.S.A) 79: 2840-44 (1982).

One problem in enzymatic synthesis of peptides is that hydrolysis is thermodynamically favored under normal conditions. Thus, the equilibrium must be shifted. Homandberg, et al., Biochemistry, 21: 3385-89 (1982) teaches that one may use a "molecular trap", that is, a molecule which has an affinity for a particular peptide of known amino acid sequence, to shift the equilibrium to favor the synthesis of such a peptide.

Varied approaches have been utilized for the purpose of generating diverse populations of peptides.

"Semisynthetic" peptides and proteins have been prepared by (1) limited proteolysis of naturally occurring polypeptides to yield a workable set of fragments, (2) chemical synthesis of an additional oligopeptide, and (3) reconstruction of synthetic and native partners. The technique is typically used to prepare analogues of naturally occurring polypeptides. Chaiken, CRC Critical Reviews in Biochemistry, 255 (Sept. 1981). Ruggeri, et al., P.N.A.S. (U.S.A.), 83: 5708-12 (Aug. 1986) prepared a series of synthetic peptides in lengths up to 16 residues that were modeled on various platelet-binding peptides. The technique used was one of solid state synthesis by chemical means, but using individual compartmentalized peptide resins to impart the desired variety. See Houghten, et al., P.N.A.S. (U.S.A.), 82: 5131-35 (1985). Yet another approach to generating a variety of peptides is through expression of mixed oligonucleotides. See Goff, et al., DNA, 6: 381-88 (1987). None of these approaches involve a balanced equilibrium between random synthesis and random degradation within a large population of peptides. What is needed is such a system of equilibria, whereby selective removal of a particular product of unknown amino acid sequence will result in net selective synthesis of that product without net synthesis of large amounts of every possible product.

The primary use for proteolytic enzymes as catalysts for peptide synthesis has previously been directed toward the synthesis of singlc, known peptide species. Several patents relate to such enzymatic synthesis of non-random peptides and, to this end, disclose and claim use of protective groups to prevent formation of other peptides. Isowa. U.S. 3,977,773 (1976) relates to use of pepsin in the enzymatic production of peptides containing three or more amino acids. Certain constraints are set on the sequence of the peptide. N- and C-terminal protective groups are broadly recited. Isowa, U.S. 4,086,136 (1978) claims the use of a thiol proteinase (e.g., papain) or a serine proteinase (e.g., subtilisin) in the enzymatic synthesis of peptides containing two or more amino acids. The claim requires that one amino acid or peptide carry an amino protective group and the other a carboxyl protective group, and the latter be one of the following: tertiary alkoxy, benzyloxy, benzylamino, and benzylhydrylamino. Isowa, U.S. 4,116,768 (1978) is drawn to production of a peptide under the action of a metalloproteinase enzyme. N- and C-terminal protective groups are broadly recited. See also Isowa, U.S. 4,119,493 (1978). Isowa, U.S. 4,165,311 (1979) is essentially directed to addition compounds for use in the production of alpha-L-aspartyl-L-phenylalanine alkyl esters (i.e., aspartame-like compounds). Isowa, U.S. 4,256,836 (1981) is drawn to the process for the production of these compounds using a protease. See also U.S. 4,521,514 (1985).

Johansen, U.S. 4,339,534 (1982; De Forened Bryggerier A/S) is broadly directed to the use of an L-specific serine or thiol carboxypeptidase, such as yeast carboxypeptidase, for enzymatic synthesis of peptides. See also Johansen, U.S. 4,806,473. Oyama, U.S. 4,521,514 (1985; Toyo Soda) is directed to a process for recovering protease. Snellman, U.S. 3,544,426 (1965) relates to synthesis of peptide chains by reaction of a carboxylic acid ester, an amine (including a peptide), a phosphoric derivative of a nucleoside, and an enzyme. Morihara, U.S. 4,320,197 and 4,320,196 relate to a semisynthesis of human insulin.

Of course, the use of proteolytic enzymes to generate peptides of exclusively pre-existing amino acid sequences through simple degradation of existing proteins is well known. For example, Matsukawa, U.S. Patent No. 3,855,196 (issued Dec. 17, 1974 to the inventors) decomposes the skeletal muscles of a cervoidae (a taxon including reindeer and caribou) with a protease into low molecular weight peptides with a molecular weight of less than 1,000. These peptides are separated from the crude digest using a gel-type molecular sieve.

Maubois, U.S. Patent No. 3,993,636 (issued Nov. 23, 1976 to the Institute National de la Recherche Agronomique) ultrafiltrated vegetable proteins using membranes with molecular weight cutoffs as low as 2,000 (col. 4, lines 38-44).

Fujimaki, U.S. 3,813,327 (issued April 9, 1974, now expired) claims obtaining an oligopeptide of MW 1,200-2,000 by hydrolyzing a protein with, e.g., subtilisin.

Pieczenik, W087/01374 further uses such uniform-sized, but variable sequence peptides derived from simple degradation of existing proteins to identify the recognition sites of antibodies. Alternatively, variable peptides are produced through chemical synthesis from amino acids, or through insertion of fractionated DNA into an expression vector and subsequent expression thereof.

Binding of peptides by macromolecules in the presence or absence of semi-permeable membranes has previously been described for a variety of purposes. Receptor binding assays are well known in the art, and the screening of peptides for ability to bind to a receptor is conventional. See, e.g., Ruggeti, supra. Westman U.S. Patent No. 3,649,457 (issued Mar. 14, 1972 to Monsanto Co.) discloses placing a soluble enzyme-polymer conjugate in an enzymatic reaction chamber having a semipermeable wall which permits passage of the enzymatic reaction products but not of the enzyme reagent. In the example, the membrane had an exclusion limit of as low as 1000 (col. 3, lines 15-18). Papain was among the enzymes suggested for use in this system (col. 9, line 48). While the reaction product must be of lower molecular weight than the enzyme-polymer conjugate, "it may be of a higher molecular weight than starting substrate, as when substrate comprises a plurality of reactants which are enzymatically reacted together to give a higher molecular weight product." (col. 13, lines 60-63). Likhite, U.S. Patent No. 3,843,444 (issued Oct. 23, 1974) discloses a membrane separation process in which a ligand in one medium is attracted across a barrier film to a receptor in a second medium. It must be emphasized that in Likhite's system, neither the receptor nor the ligand actually crosses the barrier; they are selectively collected on opposite sides thereof. (See, e.g., col. 2, lines 20-21, 25-28 and 67-70).

The present invention is directed toward satisfying the need for the generation of large, random populations of peptides of a particular size range and to concomitant selection of potentially useful peptides from such a population and synthesis of analyzable quantities of such peptides without the net production of large quantities of extraneous, random peptides. The invention fills this need by the use of scrambling reactions, i.e., a system at steady state, and preferably at a global equilibrium, between synthesis and degradation of peptides catalyzed by proteolytic enzymes, and by coupling such scrambling reactions to a means for selection and net synthesis of potentially useful peptides through perturbation of the equilibrium, in particular by binding of such peptides by a macromolecule, thus removing such peptides from the system and shifting the equilibrium to favor formation of such bound peptides. Genetically engineered proteins having enzymatic activity are also useful in promoting scrambling reactions and providing random distributions of peptides.

### BRIEF DESCRIPTION OF THE DRAWINGS

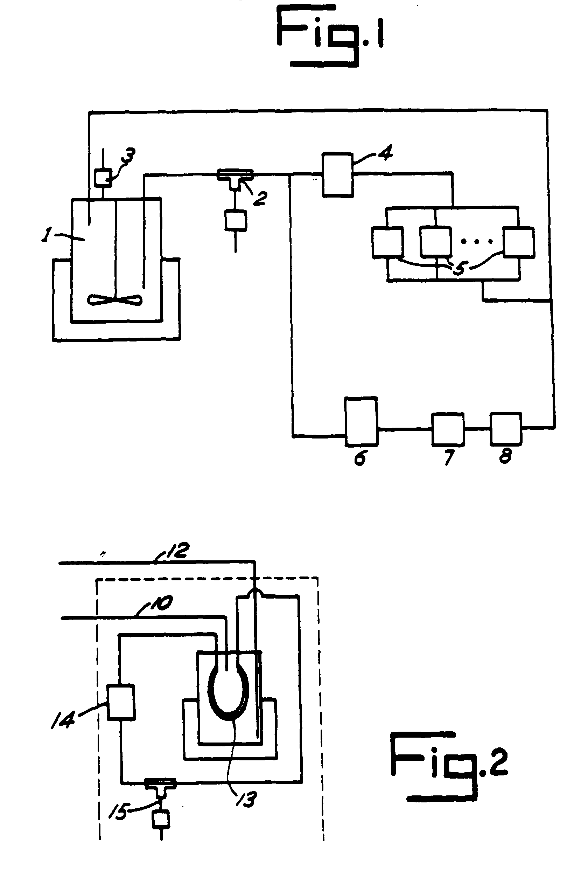

Figure 1: Schematic drawing of peptide synthesis/trapping equipment and procedures of this invention. The equipment includes a stirred, temperature controlled, reaction chamber 1, for example a system in which the proteolytic enzymes and starting peptides are placed and initiation of the scrambling reaction begun. To maintain sterility within the reaction chamber, all reagents are added to, and aliquots removed from, the pre-sterilized reaction chamber 1 by way of a micro-filtration device 2, through a three way valve. Pressure is released to the atmosphere by way of a second micro-filtration valve 3. A Gilman bacterial air vent is suitable. Suitable antibacterial agents may also be added to the reaction mixture to inhibit bacterial growth. The contents of the reaction chamber 1 in one embodiment of the invention are circulated by pump 4 through binding chamber 5. The apparatus is assembled with parallel binding chambers 5 to concurrently assay multiple macromolecular sinks and to perform comparative identification of peptides for structurally related macromolecular systems.

In a second embodiment, the contents of the reaction chamber 1 are also pumped by pump 6 through extraction chamber 7 in series, with a binding chamber 8. The series of ligand extraction chamber 7 and binding chamber 8 allows ligands of non-interest to be removed from the reaction chamber mixture by the extraction chamber 7 before presentation to the macromolecular sink of interest in the binding chamber 8. This provides a sensitive and direct method for identifying binding peptides having unique affinity for closely related macromolecular sinks. The pump 6 and extraction chamber 7 must provide a sufficiently slow flow rate, and sufficiently large surface area to deplete the reaction chamber 1 contents of those peptides having high affinity for the macromolecule present in 7.

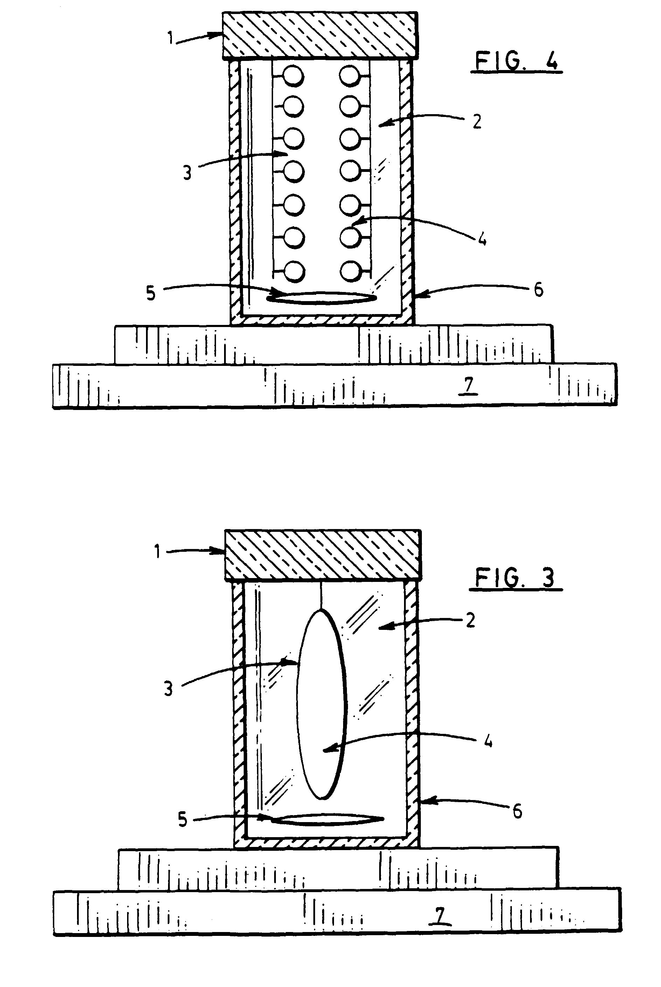

Figure 2: Detailed illustration of binding chambers 5 and 8, from Figure 1. The reaction mixture from reaction chamber 1 flows into the binding chamber 11 through line 10 and is returned by line 12.

The macromolecules are circulated through hollow fiber 13 by pump 14.

These fibers, by virtue of their semipermeable nature place the macromolecule in contact with the peptides being generated by the scrambling reaction in the reaction chamber 1 without exposing them to the catabolic environment of this mixture. A valve and micro-filtration device 15 is used on the macromolecule sink side to load the macromolecule sink, and to sample the peptide macromolecule complex. The extraction chamber 7 is similar to the binding chambers described above with the exception that the cell is designed to maximize the contact time with the reaction chamber contents in order to insure depletion of peptides having affinity for the extracted ligand before exit to the binding chamber 8. The macromolecule in 7 is sampled before the complete system reaches equilibrium in order to successfully identify peptides having unique affinity for the macromolecule in 8 relative to that in cell 7. In those cases, where different conditions (temperature, buffers, relative ratios of a.a., peptidases, etc.) are desired, different reaction chamber/binding chamber combinations are used. All binding and extraction chambers may be reused after washing.

Figure 3: Schematic of a simple semipermeable membrane-type peptide generating and screening apparatus. 1 = cap, 2 = scrambling mixture, 3 = dialysis bag, 4 = macromolecular sink, 5 = stir bar, 6 = reservoir, and 7 = magnetic stirrer. A protease protein, peptide mixture is placed in a holding reservoir (of any practical volume). The macromolecular sink (receptor) is placed in a dialysis bag (approximately one tenth the volume of the holding reservoir and having a molecular cutoff of preferably 1000-3500 daltons). The dialysis bag is immersed in the enzyme, protein, peptide mixture,and low molecular weight peptides generated in this mixture diffuse across the dialysis membrane and interact with the macromolecular sink. After a suitable period of time, the contents of the dialysis bag are removed and evaluated for peptides which have bound to the macromolecule. The binding chamber is sealed with a cap to ensure sterility of the system, and the contents of the reservoir are stirred to ensure adequate mixing of the enzyme, protein, peptide solution.

Figure 4: Schematic of a peptide generating and screening apparatus in which selective fluid communication is achieved by immobilizing both protease and macromolecular sink molecules. 1 = cap, 2 = protein and/or peptides, 3 = immobilized enzyme, 4 = immobilized macromolecular sink, 5 = stir bar, 6 = reservoir, 7 = magnetic stirrer.

In this technique a protein or protein/peptide mixture is placed in a holding reservoir (of any practical volume). The macromolecular sink (receptor) is attached to glass or sepharose beads which have been immobilized onto a rigid substrate. Similarly, the enzyme is attached to glass or sepharose beads which have been immobilized onto a separate rigid substrate. The macromolecular matrix and the enzyme matrix are immersed into the protein, peptide mixture and spatially separated from each other. Low molecular weight peptides generated as a consequence of interaction between the immobilized enzyme and the protein, peptide mixture interact with the immobilized macromolecular sink. After a suitable period of time, the rigid macromolecular sink substrate is removed and evaluated for peptides which have bound to the macromolecule. The binding chamber is sealed with a cap to ensure sterility of the system, and the contents of the reservoir are stirred to ensure adequate mixing of the protein, peptide solution.

### BRIEF SUMMARY OF THE INVENTION

A method is provided for inexpensively and rapidly producing a large and varied population of peptides, up to nearly all theoretically possible peptides in a particular size range, according to amino acid number and screening this varied population of peptides for thc presence of peptides which bind to a macromolecule or macromolecular complex and are thus potentially useful for pharmacologic or other purposes. The method further provides for the generation of analyzable (picomole to millimole) quantities of such peptides without concomitant net synthesis of such quantities of extraneous random peptides. A full showing of pharmacologic utility of a single peptide isolated from a large, random population of peptides involves the following steps: (1) generation of the peptide, (2) demonstration that a particular peptide binds specifically to a macromolecule associated with a particular physiological function, (3) isolation of the specific binding peptide, (4) determination of the structure (amino acid sequence) of the specific binding peptide, (5) large-scale synthesis of the specific binding peptide, (6) demonstration of biological activity of peptide, and (7) pre-clinical and clinical testing. Satisfactory methods currently exist for steps 3-7. What is needed is a method for generating a large, random population of peptides, screening that population for the presence of peptides which specifically bind a particular protein or macromolecule and synthesizing analyzable quantities of such peptides. The invention satisfies this need by employing (1) a "scrambling" system which utilizes one or more proteases to randomly generate large numbers of peptides of a particular size and of varied, undetermined sequences through balanced peptide synthesis and degradation, (2) a molecular trap to drive the synthetic reaction toward completion with respect to only the binding peptide, as well as to screen for the particular peptide(s) of interest, and (3) a semi-permeable barrier, covalent or matrix immobilization, or similar means of physically separating the scrambling system from the molecular trap. An object of the invention is to provide a means for inexpensive and rapid synthesis of very large random populations of peptides. Another object of the invention is to provide a method for screening such a population for the presence of peptide(s) (specific binding peptides) which bind a particular biologically important macromolecule and to be able to identify such peptide(s) by amino acid sequence. Another object of the invention is to provide a means for concomitant screening of a large, random population of peptides for the presence of peptide(s) (specific binding peptides) which bind to different biologically important macromolecules in a molecular complex whose properties are different than the same molecules in isolation.

Another object of the invention is to provide specific binding peptides of defined amino acid sequence, which having been identified by the methods of the invention, are readily produced by standard synthetic means.

### DESCRIPTION OF THE SPECIFIC EMBODIMENTS

A method is provided for synthesizing a population of peptides containing large numbers of peptides of varied sequence within a particular size range and for screening that population for the presence of one or more peptides (specific binding peptides) which specifically bind to a particular macromolecule.

The method involves a "scrambling" reaction which utilizes as starting material, one or more non-specific proteases and a mixture of size-selected peptides, isolated by gel permeation chromatography of a digest of an inexpensive natural protein, such as casein, or a mixture of natural proteins. The "scrambling" reaction relies upon the fact that enzymes are catalysts and thus can facilitate reactions in either direction. For proteolytic enzymes, this means that proteolysis (degradation of peptides to smaller peptides and amino acids) and peptide synthesis (coupling of peptides and/or amino acids to form peptides) can be facilitated by the same enzyme. The equilibrium position (synthesis versus degradation) is primarily determined by the concentration of amino acids and other peptide products. For an equilibrium position at which synthesis and degradation are balanced, peptides are continuously being randomly synthesized and then randomly degraded, and thus true "scrambling", i.e. the generation of a multiplicity of peptides containing greatly varied combinations of amino acids, occurs. Such a multiplicity of peptides may be generated by rearranging amino acids within or among peptides, and/or by digesting protein to yield peptides, followed by addition of amino acids to such peptides. Proteolytic enzymes may be employed to carry out the rearrangement or digestion and addition processes. These processes can result in a large number, even up to the theoretical maximum number, of unique peptides being produced for a peptide of any given size. For example, for an average peptide size of eight amino acids the theoretical maximum number of distinct peptides produced would be up to about 20⁸ or about 2.56 x 10¹⁰. Each of these peptides would be expected to have its own unique spatial and thermodynamic binding properties and, thus, its own unique capacity to bind to a particular target macromolecule. In comparison, the human antibody repertoire, long considered to represent the apex of binding diversity, produces a maximum of about 10⁹ unique spatial thermodynamic binding states. It should be recognized, however, that the number of distinct peptides may be less than this maximum theoretical number. This is due to the possible existence of local energy minima around which a steady state condition might develop, preventing a true global equilibrium from occurring. Considerable variation, however, would still be expected in such a system. Such variation need not necessarily approach the maximum theoretical number to be able to result in the production of useful specific binding peptides.

The method further involves producing and selecting analyzable quantities of one or more peptides (specific binding peptides) which specifically bind to a particular macromolecule. This is done by favoring the net synthesis of such peptides by removing them from the scrambling system, thus shifting the equilibrium. The peptides are removed from the system by specific binding to a specific macromolecule "trap" which may be physically separated from the "scrambling" mixture by a semi-permeable barrier which allows passage of peptides up to a particular size without allowing passage of marcomolecules, including proteases. By selectively removing a particular peptide product from the "scrambling" reaction at equilibrium, and thus preventing it from being degraded, the net synthesis of that peptide can be achieved without requiring the impracticable net synthesis of equivalent quantities of each of the other twenty-five billion (or more) peptides.

Thus, the invention allows the generation and screening of a population of peptides for the presence of peptides which specifically bind a particular macromolecule, and further allows the favored net synthesis of analyzable quantities of such peptides. By using as the "trap" a macromolecule for which binding of the peptide is desired, e.g., classical biological receptors, specific immunoglobulins, structural proteins, etc., peptides which are useful may thus be selected.

These specifically binding peptides can then be separated from the macromolecule and each other by standard procedures and identified by amino acid sequencing.

Once this identification has been made, this fully enables one skilled in the art to produce unlimited quantities of the specific binding peptide by standard synthetic means. Thus the methods of the invention enable both the production of specific binding peptides by the method of the invention, and the production of large quantities of peptides having amino acid sequences identical to specific binding peptides produced by the methods of the invention.

The method of the invention comprises the protease-catalyzed "scrambling" of peptides to yield a multiplicity of peptides comprising massive numbers of unique spatial and thermodynamic properties, coupled with net synthesis of peptides which bind to particular macromolecules and identification of said peptides according to their amino acid sequence.

The starting peptides will average from about 2 a.a. to about 15 a.a. in size. Those skilled in the art will recognize that the starting peptides may be derived from a variety of sources. Preferably, the peptides will contain all, or nearly all, of the 20 naturally-occurring amino acids. Sources of such peptides would include proteolytic digests of naturally occurring proteins as well as chemically synthesized peptides. Most preferred is the use of peptides which have been generated by proteolytic digestion of an inexpensive protein, such as casein. The most preferred size range for starting peptides is from about 7 a.a. to about 10 a.a.

The starting peptides are "scrambled" by balanced synthesis and degradation catalyzed by proteolytic enzymes. One or more proteolytic enzymes may be added to the solution of starting peptides as scrambling proceeds. The starting peptides may be present at a concentration from about 10⁻¹ M to about 10⁻⁶ M, more usually at a concentration from about 10⁻¹ M to about 10⁻⁴ M. The most preferred concentration of starting peptides is about 10⁻³ M to 10⁻⁴ M. It should be understood that not all reagents useful in the present invention will be wholly soluble under all circumstances. Therefore, molarity, for purposes of the present invention, should be construed to mean only the number of moles of material added per liter of solution, and not the number of moles actually solvated per liter of solution. The best mode contemplated for the "scrambling" reaction is to use a combination of distinct, relatively nonspecific proteases. Most preferred is the use, singly or in combination of papain, pepsin, bromelain, thermolysin. Each protease should be present at a concentration from about 0.01 weight percent of the substrate concentration to about 20 weight percent of the substrate concentration depending on molecular weight and solubility of each. Most preferred is that each enzyme will be present at about 0.5 weight percent of the substrate concentration.

Alternatively, scrambling may be accomplished by chemical, rather than by enzymatic means.

Selective net synthesis of peptides with a high binding affinity for a particular macromolecule is achieved by removing such peptides from the "scrambling" cycle by allowing them to bind to the particular macromolecule "trap" which has been separated from the "scrambling" reaction, for example, by a semi-permeable membrane which allows the free passage of the peptides, but not of the macromolecule or the proteolytic enzymes. There is no theoretical limitation on the type of macromolecule or macromolecule complex which may be used as a trap.

The use of biological and biochemical techniques has allowed the identification of numerous biomolecules whose modulation could alter a pathological state. These molecules and molecular complexes include, but are not limited to receptors, specific antibody classes, structural proteins RNA, DNA, polysaccharides and enzymes.

Examples of classical receptors which have been identified and cloned include the dopamine receptors (D1 and D2), opiate receptors, benzodiazepine receptors, adrenergic receptors (alpha-1, 2 and beta-1, 2), cholinergic (muscarinic) receptor, calcium, sodium or potassium channel receptor, glucocorticoid receptor, fibrinogen receptor and fibronectin receptor. Further examples are receptors for insulin, estrogen, LDH, progesterone, inositol triphosphate, and seratonin, among others.

Specific antibody classes include those involved in organ transplant rejection and autoimmune diseases such as rheumatoid arthritis, multiple sclerosis, Graves ophthalmopathy, psoriasis, insulindependent diabetes and systemic lupus erythematosus.

Structural proteins and enzymes include those associated with pathological viruses or bacteria such as the common cold (rhinovirus), hepatitis A, influenza, respiratory syncytial disease virus, HIV and cancer inducing viruses (e.g., HPV). Specific examples include the rhinovirus capsid protein, the reovirus hemagglutinin receptor and HIV gp120 protein. Further examples are dihydrofolate reductase and penicillinase, among others.

Nonviral structural proteins and enzymes include sickle cell hemoglobin and specific protein kinases associated with neoplasia. Further examples are collagenase inhibitors, HMG-CoA, reductase inhibitors, angiotensin converting enzyme inhibitors, renin inhibitors and thymidylate synthetase inhibitors.

In principle each macromolecule, macromolecular complex, or receptor presents a unique surface recognizable by a distinct set of spatial and thermodynamic properties and, thus, any receptor or macromolecule is theoretically capable of acting as a "trap." The concentration of macromolecule to be used as a trap is within the range from about 10⁻¹⁰ M to about 10⁻³ M. In a preferred embodiment the macromolecule concentration is from about 10⁻⁷ M to about 10⁻⁵ M. The size of the binding peptide may range from about 2 a.a. to about 15 a.a. Most preferred is a binding peptide size range from about 2 a.a. to about 10 a.a. Specific binding of the macromolecule by the peptide is defined as binding wherein the association constant is from about 10⁴ to about 10¹³ per mole. Peptides having affinities in this range are known as specific binding proteins. Most preferred are peptides with a binding affinity of at least about 10⁵ per mole, and which may cause a modulation of the activity of the bound protein.

Those skilled in the art will recognize that many configurations may be employed to separate the macromolecule "trap" from the "scrambling" reaction, such as by means of a semipermeable barrier. The only limitation is that the configuration should allow free passage of the binding peptide while preventing transmigration of the macromolecule "trap" or of any of the proteases. In other words, the barrier should allow the peptides to be in fluid communication with a specific binding macromolecule or macromolecular complex while preventing other macromolecules or macromolecular complexes (e.g., proteases) from having such communication. The barriers are typically those used in macromolecule dialysis, desalting, concentration or isolation, and are commonly characterized by selective permeability with a molecular weight cut-off of about 15kd or less. Preferred barrier materials included cellose nitrate, cellulose acetate, regenerated cellulose, polyamide, and polycarbonate polymers prepared in sheets, tubes or hollow fibers and with a permeability cutoff from about 0.2kd to about 20 kd. Most preferred is a semipermeable membrane with a permeability cutoff of about 1-3.5 kd. The spatial relationship between the semi-permeable barrier, the "scrambling" reaction, and the macromolecular "trap" may vary, but in every configuration the semi-permeable barrier will completely separate the proteolytic enzymes from the macromolecule "trap". In certain embodiments, the barrier may be used as a "bag" containing either the "scrambling" or "trap" components, or could serve as a "window" between two or more compartments containing the "scrambling" and "trap" components of the system.

The following examples are offered by way of illustration, and are not in any way limitating. Those skilled in the art will recognize that different proteolytic enzymes or combinations of proteolytic enzymes may be employed, that reaction times and concentrations of reagents may to some extent be varied, that useful peptides of different sizes may be generated, and that other means of separating the "scrambling" and "selective" systems may be employed. Although concentration values in the examples are given in terms of molarity, those skilled in the art will recognize that not all reagents will be fully in solution under all conditions. Therefore, the molarity values provided should only be construed to represent the moles of material added for each liter of solution, rather than as representing grams of actual solvated material per liter of solution. The following abbreviations are used hereafter: a.a = amino acids, W = tryptophan, E = glutamate, F = phenylalanine, Y = tyrosine, L - leucine, S = serine, G = glycine, I = isoleucine, K = lysine, V = valine, A = alanine, M = molar, g = grams, mg = milligrams, kd = kilodalton, 2-D SDS-PAGE = two-dimensional sodium dodecyl sulfate polyacrylamide gel electrophoresis.

### Example 1

### Preparation of Starting Peptides

An inexpensive protein, such as casein, bovine serum albumin, ovalbumin, lactalbumin, bovine kidney acetone powder, intestinal acetone powder, hemoglobin, edestin, chicken egg whites, cucurbit seed globulin, etc., or mixtures thereof is digested at a concentration of approximately 10⁻⁶ M with approximately 0.5% wet weight of a nonspecific protease or combination thereof, such as papain, pronase, or subtilisin. At ambient temperature (25 °C) the digestion requires about two hours. The digested material is fractionated by gel-permeation chromatography on a polyacrylamide matrix (ex. Bio-Rad®, Bio-GelM®). Fractions containing peptides of about 7-12 a.a. are collected, lyophilized and reconstituted at a concentration of about 10⁻² M for use in the scrambling reaction. Alternatively, a similar, and adequate, starting peptide distribution may be obtained by carrying out the digestion directly in the reaction chamber 1 in Figure 1. Thus, about 3L of a solution of 10⁻² M of the same inexpensive proteins or mixtures thereof are digested with approximately 0.5% wet weight of a nonspecific protease or combination thereof. The desired molecular weight distribution of small peptides having 7-12 a.a. is adjusted by either adding more protein, which will increase the average peptide length (i.e. higher concentration of a.a. and increased synthesis), or diluting the peptide mixture to decrease the peptide chain length (i.e. increased hydrolysis of existing peptides). The actual peptide size distribution is monitored by removing an aliquot of the reaction mixture from valve 2, figure 1, Example 1 and subjecting it to gel permeation chromatography using polyacrylamide gels. For both methods of making starting peptides, the equilibrium mixture may be further supplemented with an amino acid and/or small peptide whose inclusion in a given binding peptide is considered particularly desirable, or whose rate of reaction in the scrambling reaction is particularly low.

### Example 2

### Initiation of the Scrambling Reaction

To the reaction chamber of Figure 1 is added about 3L of a solution of 10⁻²M peptide fragments of 7-12 a.a. in size (Example 1), via the micro-filtration apparatus to maintain the reaction chamber sterility. This is followed by a mixture of S. griseus protease, papain, and subtilisin at about 0.5 g per 100 g of peptide substrate. The scrambling reaction is incubated with stirring at about 25°C. Alternately, this same point is reached by direct digestion of inexpensive starting proteins as given in Example 1. To assess the state of the desired global equilibrium, and thus the amount of scrambling, several techniques given in Example 3 are employed. In both cases, the peptide length is periodically monitored by removing an aliquot of the reaction mixture from the reaction chamber and subjecting it to gel permeation chromatography using polyacrylamide gels. The desired average range in peptide length, i.e. 7-12 a.a., is maintained by adding additional amino acids, peptides or protein, when greater length is desired, or by dilution with buffer when lower peptide length is desired.

### Example 3

### Qualitative Analysis of a.a. Scrambling in Peptides by Chromatographically Measuring Chemical Heterogeneity

There is no practical method to assure that true global equilibrium has been reached in the scrambling reaction. This would require proof that all 10¹³ (in the case of 10 residues) possible peptides were present in equal amounts. The most practical definition of scrambling is simply the identification of high affinity peptides whose a.a. sequence is not found in the a.a. sequences of the starting proteins in Example 1. The occurrence of a.a. scrambling in the peptides is also indicated by many other measures of free a.a. incorporation into, or peptide recombination of, existing peptides in the reaction mixturc in Example 2. For example, the following qualitative and semiquantitative analyses are useful in determining the extent to which the theoretical scrambling limit has been approached and thus an estimate of the numbers of peptides available for trapping. Initial assessment of a.a. scrambling in the peptides depends upon the fact that as scrambling occurs, chemical, as well as potential biological heterogeneity increases. Thus, the initial cleavage of a protein into fragments without scrambling produces a limited number of chemical entities, i.e. a few thousand for the small proteins being used in Example 1. Chromatography (gel filtration, reversed phase, isoelectric focusing, ion exchange, normal phase, partition, etc.) of such a digestion mixture shows structure, i.e. peaks, by virtue of the relatively high concentration of a limited number of chemical entities. As scrambling progresses, the chemical heterogeneity increases from a few thousand separate chemical entities to a maximum of about 10¹³ chemical entities for peptides 10 a.a. in length. As the chemical heterogeneity increases, there is a corresponding loss in chromatographic structure as the number of chemical entities increases and the quantity of any specific starting chemical entity decreases. In the limit, 10¹³ peptides, most differing slightly in their chromatographic behavior and each constituting only a very small equal fraction of the starting a.a. content, give a chromatogram which is simply a broad hump lacking clearly defined peaks. Intermediate stages of scrambling are characterized by partial loss of chromatographic structure. Any increase in chemical heterogeneity, as measured chromatographically relative to that of the starting peptide mix, signifies qualitatively that a.a. scrambling is occurring.

### Semi-Quantitative Analysis of a.a. Scrambling in Peptides

### Using Isotope Labeling

The amount of a.a. scrambling in the peptides can be estimated by measuring the distribution of label with time in a scrambling reaction pulse labeled with dipeptide mixtures containing all a.a. These analyses depend upon the fact that at true global equilibrium, every a.a. free in solution and every a.a. at every position in every peptide in the mixture will have equal probability of access to the pulse labels. Though it is impractical to isolate every peptide and determine that the labels are evenly distributed over all possible peptides, as a first approximation, this can be done for the bulk properties of the heterogeneous mixture of peptides. In this procedure, a mixture of dipeptides containing all twenty ³H-labeled a.a. (5 mCi, 10⁻⁷ M total, adjusted to match the a.a. distribution of the peptide reaction mixture) are added to the reaction mixture in Example 2 and the reaction allowed to incubate for a period of time. An aliquot of the reaction is removed and subjected to various forms of chromatography: gel filtration, reversed phase, isoelectric focusing, ion exchange, normal phase, partition, etc. The effluent from the chromatography matrix is analyzed for both 214 nM uv (all peptides) and ³H (those having undergone synthesis) detection. Any increase in chemical heterogeneity, as measured by ³H incorporation into new peptides derived from the unlabelled starting peptide mix, signifies qualitatively that a.a. scrambling is occurring. The greater the a.a. scrambling in the peptide mix, the closer the isotope and 214 nM UV chromatographic profiles become. At true global equilibrium (100% scrambled state) the two profiles must be uniform over the entire UV spectral range. Further definition of the amount of a.a. scrambling in the reaction mixtures can be obtained by determining the distribution of labeled a.a. at each position in the new peptide oligmers eluting from the various chromatographic procedures. At true global equilibrium, i.e. when complete a.a. scrambling in the peptides has occurred, the label will be evenly distributed over every possible a.a. compartment. Compartments are defined as being the same relative position (i.e. 3rd residue from the carboxy terminus) in a heterogeneous mix of peptides. At true global equilibrium, when truly theoretical scrambling has occurred, the ratio of labeled a.a. to unlabeled a.a. will equal the starting ratio of added label to total a.a. content of the starting peptide mix of each of these compartments. If this state has not been reached, the relativc amounts of labeled a.a. at various positions in the peptide will differ from this theoretical amount. These differences can be used to further estimate the degree to which the system has approached the true global equilibrium state. The total molar concentration of a.a. in the starting mixture is the sum of starting peptides and those added to maintain a given average peptide length (Assume a concentration of 10⁻² M total a.a. for the following calculations, the amount of labeled a.a. added as dipeptide being insignificant). The analysis is carried out by sequentially degrading the peptides in the various chromatographic fractions enzymatically, using peptidase hydrolysis, or chemically, using Edman degradation, and subsequently measuring the amount of label released as each a.a. residue is released from the peptides. The amount of scrambling, i.e. the extent to which true global equilibrium has been reached, is defined as the percentage of this observed ratio over that theoretically possible (i.e. 10⁻⁵). Values from zero to 10⁻⁵ are possiblc, reflecting the degree to which scrambling has occurred.

### Example 4

### Selective Net Synthesis and Identification of Peptides Which Bind

Reaction chamber a (See Figure 1) is brought to equilibrium as described in Examples 1-3. A mixture of all twenty ³H-labeled a.a. or small peptides containing these a.a. (5 mCi, 10 M total) are introduced into the reaction chamber and the system is allowed to distribute the labels over the peptide mixture. Approximately 10 mL of a buffered, 10⁻⁵M solution of the human platelet fibrinogen receptor (Marguerie; et al. L. Biol. Chem. 1979, 254(12), 5357) is added to the hollow fibers of one of the parallel binding chambers in the synthesis/trapping apparatus shown in Figure 1. The total system is allowed sufficient time to reach its new equilibrium state (or steady state), or for that time necessary to produce specific binding peptides of interest. After a given time, an aliquot of the solution from the hollow fibers, containing the platelet fibrinogen receptor and bound peptides, is removed and subjected to ultrafiltration to separate the receptor peptide complex from non-specific peptides present in the solution (the latter are returned to the reaction chamber). Separation of bound peptides from non-specific peptides is completed by washing the filtrate three times with phosphate buffer using conventional ultrafiltration equipment. This leaves a platelet fibrinogen receptor complexed to peptides whose affinities are greater than about Ka=10⁶. The platelet fibrinogen receptor complex, thus isolated, is heat denatured (100°C for one hour) to release the bound peptides. These peptides, having specificity for the fibrinogen receptor, are then separated from the denatured receptor by ultrafiltration. The solution of unique peptides for the fibrinogen receptor are fractionated using standard chromatographic methods in combination or separately, i.e. reversed phase HPLC, isoelectric focusing, adsorption chromatography, partition chromatography, ion exchange, etc. Detection of the peptides is made using a scintillation counter. The individual peptides are then sequenced by standard peptide sequencing techniques, synthesized, and evaluated for antiplatelet activity by conventional procedures. Those peptides, or synthetic modifications thereof, expressing antiplatelet activity are drug candidates for critical care treatment of various cardiovascular diseases.

### Example 5

### Selective Net Synthesis and Identification of Peptides Which Bind to Renin

A buffered solution of commercially available renin (10 mL, 10⁻ ³ M) is added to the hollow fibers of a second parallel binding chamber in the synthesis/trapping apparatus shown in Figure 1, and the system allowed to incubate for a period of time necessary to produce high affinity peptides of interest. This analysis can be conducted at the same time as that of Example 4, since any competition for a particular peptide (or peptide fragment used in the synthesis of a particular peptide) between the two macromolecular sinks may only lengthen the time to reach a new global equilibrium (or new steady state), not its final state. Procedures identical to those described for Example 4 concurrently identify peptides having high affinity for renin. Such peptides having binding specificity for renin, or synthetic modifications thereof, are potential candidates for inhibiting renin function and thus for the treatment of hypertension.

### Example 6

### Selective Net Synthesis and Identification of Peptides Which Bind to Collagenase IV

A buffered solution of collagenase IV, (10 mL, 10⁻³ M) (Salo J. Biol. Chem. 1983, 258, 2058) whose enzymatic activity appears to be important to tumor metastasis, is added to the hollow fibers of a third parallel binding chamber in the synthesis/trapping apparatus shown in Figure 1, and the system allowed to incubate for a period of time necessary to produce specific binding peptides for collagenase IV. Procedures identical to those described for Example 4 concurrently identify peptides having high affinity for collagenase IV. The peptides having this property are screened for their ability to inhibit collagenase enzymatic activity using standard assays procedures for this enzyme. Those peptides expressing collagenase IV inhibitory activity, or synthetic modifications thereof, are potential candidates as injectables for reducing the likelihood of tumor metastasis.

### Example 7

### Selective Net Synthesis and Identification of Peptides Which Bind to Sickle Hemoglobin but not to Normal Hemoglobin

The scrambling/trapping equipment assemblage lends itself to comparative identification of peptides having high affinity for closely related macromolecular systems. By way of example, hemoglobin and sickle hemoglobin, which differ by a single a.a., may be concurrently evaluated for the identification of high affinity peptides. A buffered solution of freshly prepared hemoglobin (10 mL 10⁻³ M), and sickle hemoglobin at an identical concentration, are separately loaded into the hollow fibers of two additional parallel binding chambers in the synthesis/trapping apparatus shown in Figure 1, and the system allowed to incubate for a period of time necessary to produce high affinity peptides of interest. Comparison of the chromatography profiles of the high affinity peptides from the two hollow fibers containing the hemoglobin and sickle hemoglobin define, by difference, those peptides which bind to sickle hemoglobin but not to normal hemoglobin. Peptides having unique affinity for sickle hemoglobin, or their synthetic derivatives, are potential candidates for inhibiting the sickling process and, thus, for the treatment of sickle cell anemia. This result is also accomplished by using the series, ligand extraction/binding chambers 7 in Figure 1 where hemoglobin is loaded into the extraction chamber 7, and the sickle hemoglobin into the assay chamber 8.

### Example 8

### Pepsin-catalyzed Digestion of Protein to Yield Peptides and Addition of Amino Acids Thereto

Casein (0.5mM) and pepsin (0.25mM) were incubated together with tritium-labelled WE dipeptide in 50mM potassium phosphate buffer (pH 5.0) at 22°C. Samples were then subjected to reverse phase HPLC to separate the peptides and analyze them for tritium content. After 1, 5 and 7 days of reaction time, tritium was incorporated into multiple new peptides, indicating that pepsin can both digest the casein to yield peptides and then add amino acids from the dipeptides to these peptides.

### Example 9

### Pepsin or Papain-Catalyzed Synthesis of Multiple Peptides from Dipeptides

Tritium-labelled WE dipeptide (50mM, 0.9 uCi) was incubated with either pepsin (0.25mM) or papain (0.25mM) in 50mM potassium phosphate (pH 5.0) at 22°C. When papain was used, it was first activated at 22°C for 2 hours in 50mM KCN, 15mM EDTA, 60mM acetate in water. Peptides were separated and analyzed as in Example 8. Formation of multiple new peptides was observed after I or 5 days of reaction, demonstrating that both papain and pepsin are capable of synthesizing new peptides.

### Example 10

### Generation of a Multiplicity of Peptides By a Protease Mixture

Dipeptides EW, WE and FY were incubated with pepsin and papain under the conditions described in Example 9. After I day multiple new peptide products were produced in numbers exceeding the sum of peptides produced from individual dipeptides.

### Example 11

### Protease-catalyzed Rearrangement of Amino Acids Within and Among Peptides

The tripeptide YYF was incubated with pepsin under conditions described in Example 8. Alternatively, tripeptide YYF (25mM) and tetrapeptide VAAF (25mM) were coincubated with pepsin (0.25mM). Samples were analyzed for peptide products at hourly intervals between I and 24 hours. Monopeptides, dipeptides, tripeptides, tetrapeptides and pentapeptides were present in all samples, with species having varied amino acid sequences being present. Both inter and intrapeptide amino acid exchange was observed. The product profile changed dramatically between 1 and 24 hours. This demonstrates that pepsin catalyzes scrambling of peptides, i.e., rearranging of amino acids within and among peptides in a dynamic equilibrium between hydrolysis and formation of peptide bonds.

### Example 12

### Disproportionate Increase in Diversity of Multiplicity of Peptides Generated, Relative to Diversity of Reactants

Di- or tripeptides (50 and 25mM respectively) LY and LSF, WE and LSF, WE and GGI, or WE and GLY were incubated with thermolysin (0.lmM) at 22°C in 50mM potassium phosphate (pH 8.0). Alternatively, di- and tripeptides LW (50mM) and KYK (25mM) or WF (50mM) and with 0.lmM bromelain in 50mM potassium phosphate (pH 8.0), 5mM 2-mercaptoethanol. After 24 hours multiple new peptides are formed that are absent when only the dipeptide or tripeptide alone is incubated with thermolysin. This demonstrates that more complex mixtures of reactant peptides produce disproportionately more complex peptide products.

### Example 13

### Equilibrium Shifting by a Macromolecular Sink

Casein (0.5mM) was incubated with pepsin (0.25mM) in 50mM potassium phosphate (pH 6.3), 5mM EGTA, 2mM MgCl₂ at 22°C in the presence of a dialysis bag having a 1000 dalton cut-off and containing iso-osmolar buffer with or without fibrinogen (0.03mM). After 1 to 93 hours low molecular weight products are detectable in the dialysis bag in either case. The products in the dialysis bag were different in the presence of fibrinogen than its absence, and the quantity of at least one product increased as a function of time in the presence of fibrinogen. This demonstrates that (I) low molecular weight products resulting from proteolytic digestion of casein can diffuse across a semi-permeable barrier, and (2) the presence of a macromolecular sink across the semi-permeable barrier from the scrambling reaction can shift the equilibrium of the scrambling reaction to favor synthesis of specific products.

### Example 14

### Bound Complex Formation and Specific Binding Peptide Identification Using Various Reactant Peptides, Proteases and Macromolecular Sinks

Dipeptide FY (50mM) was incubated with pepsin (0.25mM) in 50mM potassium phosphate (pH 5.0) at 22°C for 24 hours. The reaction mixture was then placed outside a dialysis bag with a molecular weight cutoff of 1000 daltons. The dialysis bag contained I ml of 50mM bovine serum albumin (BSA) in iso-osmolar buffer. After 5 days, aliquots from inside the dialysis bag were filtered through a 10,000 dalton cutoff filter membrane, which was then washed with iso-osmolar buffer. The membrane retentate was resuspended in iso-osmolar buffer and denatured at 100°C for 15 minutes to separate the specific binding peptide from the bound complex then filtered through a 1000 dalton cutoff filter. Filtrate was subjected to reverse phase HPLC for peptide separation and analysis, using peptide standards. When fibrinogen or casein was substituted for BSA as the macromolecular sink, different specific binding peptides were obtained.

### Example 15

### Transportation of a.a. from Proteins into Scrambled Peptides

Dipeptide WE (50mM) and tripeptide LSF (25mM) were coincubated with thermolysin (0.lmM) under conditions described in example 12. After five days a multiplicity of new larger peptides of various sequence were obtained. One such peptide contained isoleucine, presumably from the thermolysin. Thus the smaller peptides, as well as the protein are capable of donating a.a. that can be incorporated into larger scrambled peptides.

## Claims

1. A method of identifying peptides which bind specifically to a predetermined macromolecular target, comprising the steps of:
(a) subjecting a mixture initially comprising a plurality of starting peptides to conditions which include exposure to one or more proteolytic enzymes and under which the starting peptides and derivatives thereof can undergo both random degradation into smaller peptide and free amino acid derivatives, and random recombination of the starting peptides and/or derivatives of the starting peptides into new peptides, whereby the component amino acids of the starting peptides of said mixture as a result of the activity of the proteolytic enzyme or enzymes are scrambled to generate a diverse population of scrambled peptides of different sequences, said scrambled peptides not being characterized by a predetermined amino acid sequence;
(b) allowing the scrambled peptides to contact a specific predetermined target and to compete with each other to bind therewith to form a bound specific binding peptide-target complex;
(c) protecting, by physically separating them from the proteolytic enzyme or enzymes, only those peptides bound to the target from further scrambling;
(d) recovering the specific binding peptide from the peptide-target complex; and
(e) sequencing the peptide.

2. A method according to claim 1, wherein one or more of the proteolytic enzyme is/are nonspecific.

3. A method according to claim 2 wherein the nonspecific enzyme or enzymes is/are selected from the group consisting of papain, pepsin, bromelain, thermolysin, pronase, subtilisin and S. griseus proteinase.

4. A method according to any of the preceding claims wherein the protection from further scrambling is provided by interposing a semipermeable membrane between said scrambling reagents and said target, said membrane being permeable to said peptides and impermeable to said target and said scrambling reagents, the semipermeable membrane having a permeability cutoff of, preferably, about 1-3.5 kD.

5. The method of any of claims 1-4, further comprising monitoring the peptide size distribution from time to time while the scrambling reaction is in progress, and, optionally, adjusting the peptide size distribution during the course of the scrambling reaction by adding more amino acids, peptides or protein, or by diluting the reaction mixture.

6. The method of any of claims 1-5 wherein the average length of the peptides of the starting mixture is in the range of 7 to 12 amino acids, more preferably 7-10 amino acids.

7. The method of any of claims 1-6 wherein substantially all of the twenty genetically encoded amino acids are represented in the peptides of the starting mixture.

8. A method of any of claims 1-7 wherein the specific binding macromolecule or macromolecular complex is fibrinogen receptor, sickle cell hemoglobin, collagenase IV, or renin.

9. The method of any of claims 1-8 in which the starting peptides are obtained by cleaving one or more proteins, molecules of which optionally are present in the starting mixture.

10. The method of any of claims 1-9 in which the starting mixture further comprises free amino acids.

11. An apparatus for use in a method according to any of the preceding claims, the apparatus comprising a scrambling reaction compartment, a trap compartment, and a semipermeable membrane separating said scrambling reaction compartment from said trap compartment, said scrambling reaction compartment holding a mixture of peptides of at least partially indeterminate amino acid sequences and one or more scrambling reagents comprising one or more proteolytic enzymes, said compartment comprising inlet means communicating with said scrambling reaction compartment for introducing amino acids, peptides, diluent or additional scrambling reagents into said scrambling reaction compartment, said trap compartment comprising one or more predetermined targets potentially bindable by a peptide, said semipermeable membrane permitting the passage of peptides but not of said target or said scrambling reagents between compartments.

## Patentansprüche

1. Verfahren zum Identifizieren von Peptiden, die auf spezifische Wcise an ein vorbestimmtes makromolekulares Ziel binden, wobei das Verfahren die folgenden Schritte umfaßt:
(a) ein Gemisch, das anfänglich eine Vielzahl von Ausgangspeptiden enthält, wird Bedingungen unterworfen, die das Einwirkenlassen von einem oder mehreren proteolytischen Enzymen einschließen, und unter denen die Ausgangspeptidc und deren Derivate sowohl einem statistischen Abbau in kleinere Peptid- und freie Aminosäurederivate als auch einer statistischen Rekombination der Ausgangspeptide und/oder Derivate der Ausgangspeptide in neue Peptide unterzogen werden können, wodurch die Komponenten-Aminosäuren der Ausgangspeptide des Gemischs infolge der Aktivität des proteolytischen Enzyms oder der proteolytischen Enzyme untereinander gemischt werden unter Bildung einer verschiedenartigen Population von vermischten Peptiden mit verschiedenen Sequenzen, wobei die vermischten Peptide nicht durch eine vorbestimmte Aminosäuresequenz gekennzeichnet sind;
(b) die vermischten Peptide werden mit einem spezifischen vorbestimmten Ziel in Kontakt treten gelassen und miteinander um die Bindung damit konkurrieren gelassen, um einen gebundenen spezifischen Bindungspeptid-Ziel-Komplex zu bilden;
(c) nur diejenigen Peptide, die an das Ziel gebunden sind, werden, indem sie auf physikalische Weise von dem proteolytischen Enzym oder den proteolytischen Enzymen abgetrennt werden, vor einem weiteren Vermischen geschützt;
(d) das spezifische Bindungspeptid wird aus dem Peptid-Ziel-Komplex wiedergewonnen; und
(e) das Peptid wird sequenziert.

2. Verfahren nach Anspruch 1, wobei eines oder mehrere der proteolytischen Enzyme unspezifisch ist.

3. Verfahren nach Anspruch 2, wobei das unspezifische Enzym oder die unspezifischen Enzyme ausgewählt wird/werden aus Papain, Pepsin, Bromelain, Thermolysin, Pronase, Subtilisin und der S. griseus-Proteinase.

4. Verfahren nach einem der vorhergehenden Ansprüche, wobei der Schutz vor einem weiteren Vermischen bereitgestellt wird, indem eine semipermeable Membran zwischen die Vermischungsreagenzien und das Ziel gebracht wird, wobei die Membran gegenüber den Peptiden permeabel und gegenüber dem Ziel und den Vermischungsreagenzien impermeabel ist, und wobei die semipermeable Membran ein Permeabilitäts-Rückhaltevermögen von vorzugsweise etwa 1 bis 3,5 kD besitzt.

5. Verfahren nach einem der Ansprüche 1 bis 4, weiterhin umfassend das Verfolgen der Peptidgrößenverteilung von Zeit zu Zeit während des Fortschreitens der Vermischungsreaktion, und, wahlweise, das Einstellen der Peptidgrößenverteilung während des Ablaufs der Vermischungsreaktion durch Zusetzen von mehr Aminosäuren, Peptiden oder Protein oder durch Verdünnen des Reaktionsgemischs.

6. Verfahren nach einem der Ansprüche 1 bis 5, wobei die durchschnittliche Länge der Peptide des Ausgangsgemischs in dem Bereich von 7 bis 12 Aminosäuren, stärker bevorzugt von 7 bis 10 Aminosäuren, liegt.

7. Verfahren nach einem der Ansprüche 1 bis 6, wobei im wesentlichen alle der zwanzig auf genetische Weise kodierten Aminosäuren in den Peptiden des Ausgangsgemischs vertreten sind.

8. Verfahren nach einem der Ansprüche 1 bis 7, wobei das spezifische bindende Makromolekül oder der spezifisch bindende makromolekulare Komplex der Fibrinogenrezeptor, Sichelzell-Hämoglobin, Kollagenase IV oder Renin ist.

9. Verfahren nach einem der Ansprüche 1 bis 8, in dem die Ausgangspeptide durch Spalten von einem oder mehreren Proteinen erhaltcn werden, deren Moleküle wahlweise in dem Ausgangsgemisch vorkommen.

10. Verfahren nach einem der Ansprüche 1 bis 9, in dem das Ausgangsgemisch weiterhin freie Aminosäuren enthält.

11. Vorrichtung zur Verwendung in einem Verfahren gemäß einem der vorhergehenden Ansprüche, wobei die Vorrichtung einen Vermischungsreaktionsbereich, einen Abfangbereich und eine semipermeable Membran umfaßt, die den Vermischungsreaktionsbereich von dem Abfangbereich trennt, wobei der Vermischungsreaktionsbereich ein Gemisch von Peptiden mit zumindest teilweise unbestimmten Aminosäuresequenzen und ein oder mehrere Vermischungsreagenz/ien faßt, das/die ein oder mehrere proteolytische Enzyme umfäßt/umfassen, wobei dieser Bereich Mittel zum Einlaß umfaßt, die mit dem Vermischungsreaktionsbereich zur Einführung von Aminosäuren, Peptiden, einem Verdünnungsmittel oder zusätzlichen Vermischungsreagenzien in den Vermischungsreaktionsbereich in Verbindung stehen, wobei der Abfangbereich eine oder mehrere vorbestimmte Ziele, die potentiell durch ein Peptid gebunden werden können, umfaßt, wobei die semipermeable Membran die Passage von Peptiden, aber nicht dcs Ziels oder der Vermischungsreagenzien, zwischen den Bereichen erlaubt.

## Revendications

1. Procédé d'identification des peptides qui se lient spécifiquement à une cible macromoléculaire prédéterminée, comprenant les étapes consistant à:
(a) soumettre un mélange comprenant initialement plusieurs peptides de départ à des conditions qui incluent l'exposition à une ou plusieurs enzymes protéolytiques et sous lesquelles les peptides de départ et leurs dérivés peuvent subir à la fois une dégradation statistique en plus petits peptides et en dérivés acides aminés libres, et une recombinaison aléatoire des peptides de départ et/ou des dérivés des peptides de départ en nouveaux peptides, ce qui fait que les acides aminés composants des peptides de départ dudit mélange sont brouillés à la suite de l'activité de la ou des enzyme(s) protéolytique(s) pour engendrer une population diverse de peptides brouillés de séquences différentes, lesdits peptides brouillés n'étant pas caractérisés par une séquence prédéterminée d'acides aminés;
(b) permettre aux peptides brouillés d'entrer en contact avec une cible prédéterminée spécifique et d'entrer en compétition entre eux pour se lier avec elle afin de former un complexe peptide de liaison spécifique lié-cible;
(c) ne protéger, en les séparant physiquement de la ou des enzyme(s) protéolytique(s), que les peptides liés à la cible contre tout brouillage ultérieur;
(d) recueillir le peptide de liaison spécifique à partir du complexe peptide-cible; et
(e) séquencer le peptide.

2. Procédé selon la revendication 1, dans lequel une ou plusieurs des enzymes protéolytiques est/sont non-spécifique(s).

3. Procédé selon la revendication 2 dans lequel la ou les enzymes non spécifique(s) est/sont choisie(s) dans le groupe constitué par la papaïne, la pepsine, la bromélaïne, la thermolysine, la pronase, la subtilisine et la protéinase de S. griseus.

4. Procédé selon l'une quelconque des revendications précédentes, dans lequel la protection contre tout brouillage ultérieur est fournie par l'interposition d'une membrane semi-perméable entre lesdits réactifs de brouillage et ladite cible, ladite membrane étant perméable auxdits peptides et imperméable à ladite cible et auxdits réactifs de brouillage, la membrane semi-perméable ayant un seuil de perméabilité qui est de préférence d'environ 1-3,5 kD.

5. Procédé de l'une quelconque des revendications 1-4, dans lequel en outre on contrôle la répartition de taille des peptides de temps à autre au fur et à mesure du déroulement de la réaction de brouillage et, facultativement, on ajuste la répartition de la taille des peptides au cours de la réaction de brouillage en ajoutant des acides aminés, des peptides ou des protéines supplémentaires, ou en diluant le mélange réactionnel.

6. Procédé de l'une quelconque des revendications 1-5 dans lequel la longueur moyenne des peptides du mélange de départ est dans un intervalle de 7 à 12 acides aminés, de préférence de 7 à 10 acides aminés.

7. Procédé de l'une quelconque des revendications 1-6 dans lequel sensiblement la totalité des vingt acides aminés encodés génétiquement est représentée dans les peptides du mélange de départ.

8. Procédé de l'une quelconque des revendications 1-7 dans lequel la macromolécule ou le complexe macromoléculaire de liaison spécifique est un récepteur de fibrinogène, de l'hémoglobine de cellules falciformes, de la collagénase IV, ou de la rénine.

9. Procédé de l'une quelconque des revendications 1-8 dans lequel on obtient les peptides de départ en clivant une ou plusieurs protéines dont des molécules sont facultativement présentes dans le mélange de départ.

10. Procédé de l'une quelconque des revendications 1-9 dans lequel le mélange de départ comprend en outre des acides aminés libres.

11. Appareil pour utilisation dans un procédé selon l'une quelconque des revendications précédentes, l'appareil comprenant un compartiment de réaction de brouillage, un compartiment piège, et une membrane semi-perméable séparant ledit compartiment de réaction de brouillage d'avec ledit compartiment piège, ledit compartiment de réaction de brouillage contenant un mélange de peptides de séquences d'acides aminés au moins partiellement indéterminées et un ou plusieurs réactifs de brouillage comprenant une ou plusieurs enzymes protéolytiques, ledit compartiment comprenant un organe d'entrée communiquant avec ledit compartiment de réaction de brouillage pour introduire des acides aminés, des peptides, un diluant ou des réactifs de brouillage supplémentaires dans ledit compartiment de réaction de brouillage, ledit compartiment piège comprenant une ou plusieurs cibles prédéterminées potentiellement liables par un peptide, ladite membrane semi-perméable permettant le passage de peptides mais non de ladite cible ni desdits réactifs de brouillage entre les compartiments.
